# EUROPÄISCHE PATENTSCHRIFT

(11) **EP 3 181 188 B1**
(45) Veröffentlichungstag und Bekanntmachung des Hinweises auf die Patenterteilung: **20.06.2018**
(21) Anmeldenummer: 16199801.8
(22) Anmeldetag: 21.11.2016
(51) Int. Cl.: A61N 1/05

(54) **DEHNBARE ELEKTRODE**
EXTENDABLE ECLECTRODE
ÉLECTRODE EXTENSIBLE

(30) Priorität: 15.12.2015 DE 102015121817
(43) Veröffentlichungstag der Anmeldung: 21.06.2017
(73) Patentinhaber: BIOTRONIK SE & Co. KG, 12359 Berlin (DE)
(72) Erfinder: BIHLER, Eckardt, 8406 Winterthur (CH); HAUER, Marc, 8610 Uster (CH)
(74) Vertreter: Galander, Marcus

(56) Entgegenhaltungen:
- WO-A1-94/17852
- US-A- 5 522 874
- US-A1- 2006 074 470
- US-A1- 2008 027 524
- US-A1- 2008 039 896
- US-A1- 2012 158 097
- US-A1- 2012 158 109

## Beschreibung

Die Erfindung betrifft eine implantierbare Elektrode mit einem Kontaktelement zur Kontaktierung von Körpergewebe, beispielweise Muskelzellen des Herzens oder Nervenenden, und mit einem Verbindungsabschnitt zur Verbindung eines medizinischen Geräts, etwa ein Schrittmacher, mit dem Kontaktelement. Ferner betrifft die Erfindung ein Verfahren zum Herstellen einer implantierbaren Elektrode mit einem Kontaktelement zur Kontaktierung von Körpergewebe, und mit einem Verbindungsabschnitt zur Verbindung eines medizinischen Geräts mit dem Kontaktelement.

Implantierbare Elektroden und Verfahren zu deren Herstellung sind allgemein bekannt. Quer zu ihrer Längsachse sind implantierbare Elektroden oftmals flexibel und leicht biegbar. Entlang der Längsachse sind die Elektroden jedoch im Wesentlichen nicht dehnbar, wobei bekannte Elektroden entlang der Längsachse beispielsweise weniger als 1 % dehnbar sind.

Die Dokumente US5522874A, US20120158109A1, US20120158097A1, WO1994017852A1, US20080027524A1, US20080039896A1 und US20060074470A1 beschreiben Implantierbare Elektroden mit mindestens einem Kontaktelement zur Kontaktierung von Körpergewebe, und mit einem Leiter zur Verbindung eines medizinischen Gerätes mit dem mindestens einen Kontaktelement wobei sich der Leiter zumindest teilweise um die Längsachse der Elektrode herum windet.

Der Erfindung liegt daher die Aufgabe zugrunde, eine implantierbare Elektrode und ein Verfahren zum Herstellen einer implantierbaren Elektrode bereit zu stellen, wobei die implantierbare Elektrode entlang ihrer Längsachse zumindest abschnittsweise dehnbar ist.

Für die eingangs genannte Elektrode ist die Aufgabe dadurch gelöst, dass sich der Verbindungsabschnitt zumindest teilweise um die Längsachse der Elektrode herum windet. Für das eingangs genannte Verfahren ist die Aufgabe dadurch gelöst, dass der Verbindungsabschnitt zumindest teilweise um eine Achse herum gewunden wird. Dadurch, dass der Verbindungsabschnitt zumindest teilweise gewunden ausgeformt ist, läßt sich die Elektrode und insbesondere deren Verbindungsabschnitt einfach entlang der Längsachse dehnen, also auseinander ziehen, selbst wenn der Verbindungsabschnitt an sich nicht elastisch dehnbar ist.

Die erfindungsgemäße Lösung kann durch verschiedene, jeweils für sich vorteilhafte, und, sofern nicht anders ausgeführt, beliebig miteinander kombinierbare Ausgestaltungen weiter verbessert werden. Auf diese Ausgestaltungsformen und die mit ihnen verbundenen Vorteilen ist im Folgenden eingegangen.

So kann der Verbindungsabschnitt mindestens eine Teilwindung, wenigstens eine ganze Windung oder mehr als eine Windung aufweisen. Je mehr Windungen der Verbindungsabschnitt aufweist, desto leichter lässt sich der Verbindungsabschnitt entlang der Längsachse auseinander ziehen. Ferner lässt sich mit steigender Windungsanzahl auch die maximal mögliche Verlängerung des Verbindungsabschnitts entlang der Längsachse vergrößern.

Beispielsweise ist der Verbindungsabschnitt zumindest abschnittsweise helixförmig ausgebildet. Die Helix ist eine vergleichsweise regelmäßige Struktur, die sich einfach herstellen lässt.

Eine den gewundenen Teil des Verbindungsabschnitts umhüllende Form entspricht im Wesentlichen einem Zylinder, wenn die Längsachse gerade verläuft und die Elektrode nicht quer zur Längsachse gebogen ist. Ist die Elektrode gebogen, entspricht die umhüllende Form einem Schlauch. Durch die im Wesentlichen zylindrisch oder schlauchartig ausgeformte Umhüllende ragen keine Teile des Verbindungsabschnitts über andere Teile des Verbindungsabschnitts hervor, sodass der Verbindungsabschnitt einfach implantierbar ist.

Der Verbindungsabschnitt ist als ein um die Längsachse herum gewundenes flaches Band ausgeformt. Das flache Band kann ferner das mindestens eine Kontaktelement tragenden Kontaktabschnitt der Elektrode ausformen. Ein flaches Band lässt sich einfacher winden als beispielsweise ein rundes Kabel, das womöglich auf einer Innenseite der Windung Falten bilden kann.

Der Verbindungsabschnitt ist flächig ausgebildet und die zumindest eine Verbindungsleitung ist in einen flächigen Mantel eingebettet. Der flächige Mantel wurde mit der zumindest einen eingebetteten Verbindungsleitung in die gewundene Form gebracht. Zum Beispiel kann der Mantel als ein Flachbandkabel ausgebildet werden, wobei die zumindest eine Verbindungsleitung zwischen zwei elektrisch isolierenden Folien, insbesondere thermoplastische Polymerfolien, zum Beispiel aus einem Flüssigkristall-Polymer, angeordnet wird. Insbesondere kann eine der Folien auf einer ihrer Seiten mit der zumindest einen Verbindungsleitung versehen werden. Auf der der Verbindungsleitung gegenüberliegenden Seite dieser Folie kann die Folie mit dem wenigstens einen Kontaktelement versehen sein oder werden. Die Verbindungsleitung und/oder das Kontaktelement können additiv oder subtraktiv auf die Folie aufgebracht werden. Um die Verbindungsleitung und das Kontaktelement miteinander in Verbindung bringen zu können, kann eine Durchkontaktierung vorgesehen werden, die als ein sogenanntes metallisiertes Via ausgebildet sein kann. Ein Via ist beispielsweise eine die beiden Seiten der Folie miteinander verbindende metallisierte Öffnung in der Folie.

Der Verbindungsabschnitt und insbesondere dessen gewundener flächiger Mantel kann breite und schmale Seiten aufweisen, wobei eine der breiten Seiten eine von der Längsachse weg weisende Außenseite ist. Die Außenseite ist vorzugsweise flach und ermöglicht, dass insbesondere der gewundene Teil des Verbindungsabschnitts bei der Implantation einfach an Gewebe anliegend gleiten kann, ohne mit diesem zu verhaken. Diese Elektrode ist also einfach implantierbar.

Um die Windung dauerhaft bereitstellen zu können, kann die Außenseite größer sein als eine der Längsachse zugewandte Innenseite des Verbindungsabschnitts und insbesondere des gewundenen flächigen Mantels.

Die Außenseite kann stärker plastisch verformt und beispielsweise gestreckt sein als die Innenseite. Die stärkere Streckung der Außenseite lässt sich einfach mechanisch erzeugen.

Die Außenseite kann mechanisch gedehnt und dabei thermisch behandelt worden sein, um die größere Außenseite zu erzeugen. Die Kombination aus mechanischer Dehnung oder Streckung und thermischer Behandlung ermöglicht die Vergrößerung und beispielsweise plastische Dehnung der Außenseite bei geringen mechanischen Kräften, sodass eine Beschädigung der Elektrode vermieden und Produktionsausschuss verringert wird.

Alternativ oder zusätzlich zur vergrößerten Ausbildung der Außenseite kann der Verbindungsabschnitt und insbesondere dessen gewundener flächiger Mantel mit einer eine mechanischen Spannung auf den Verbindungsabschnitt ausübenden Schicht versehen sein, um die Windung zu erzeugen. Ist die Schicht auf der Außenseite des gewundenen Verbindungsabschnitts aufgetragen, so kann die Schicht ausgebildet sein, eine Druckspannung zu erzeugen. Eine auf die Innenseite des gewundenen Verbindungsabschnitts aufgebrachte Schicht kann ausgebildet sein, eine Zugspannung zu erzeugen. Die Außenseite und/oder die Innenseite können jeweils mit einer Schicht versehen sein.

Der Kontaktabschnitt kann ungewunden ausgeformt sein und in einem unverformten Zustand, in dem er beispielsweise auf einer ebenen Fläche liegt, im Wesentlichen eben ausgebildet sein.

Der Verbindungsabschnitt ist um einen zylindrischen Kern herum gewunden. In der gewundenen Form können einzelne oder alle Windungen an wenigstens einer Nachbarwindung befestigt sein, um die gewundene Form dauerhaft beizubehalten. Ferner sind einzelne oder alle Windungen am Kern befestigt. Um die Elektrode und insbesondere deren Verbindungsabschnitt mit einem Kern, der an den Windungen fixiert ist, in die Länge ziehen zu können, ist der Kern entlang der Längsachse der Elektrode flexibel.

Der Kern ist zylindrisch, vorzugsweise hohlzylindrisch und beispielsweise schlauchförmig ausgebildet. Der Kern verhindert, dass der Mantel bei einem womöglich zu starkem Biegen, also bei einem zu kleinen Biegeradius, einknickt und entstehende Knickkanten oder - ecken die Implantation der Elektrode erschweren.

Der Mantel kann stoffschlüssig und zum Beispiel durch Verschmelzen mit dem Kern verbunden sein, wodurch die Verbindung zwischen Mantel und Kern ohne weitere Hilfsmittel, beispielsweise Kleber oder Nähte, ausgebildet sein kann.

Der Kern kann mit einem sich entlang der Längsachse der Elektrode durchgängig durch den Kern erstreckenden Lumen ausgebildet sein, damit die Elektrode beispielsweise mit Hilfe eines Führungsdrahtes, der die Elektrode bei der Implantation führt, implantierbar ist. Ist die Elektrode ohne Kern ausgebildet, kann der gewundene Mantel des Verbindungsabschnittes das Lumen bereitstellen.

Das Material des Mantels kann ein thermoplastisches Polymer, beispielsweise ein Flüssigkristall-Polymer sein. Der Kern kann beispielsweise aus Glas oder ebenfalls aus einem Polymer, zum Beispiel aus einem Flüssigkristall-Polymer, gefertigt sein und sogar daraus bestehen. Die Verbindungsleitung besteht zum Beispiel aus Gold oder aus einer Goldlegierung. Das zumindest eine Kontaktelement kann aus Gold, aus einer Goldlegierung oder zum Beispiel aus einer Platin-Iridium-Legierung gefertigt sein.

Die Folie kann mit dem zumindest einen Kontaktelement und der mindestens einen Verbindungsleitung um den Kern herum gelegt werden. Alternativ oder zusätzlich kann eine weitere Folie auf die Seite der Folie, die die Verbindungsleitung aufweist, aufgelegt werden. Insbesondere wenn die Folien thermoplastische Polymerfolien sind, können die beiden Folien thermisch dauerhaft miteinander verbunden und beispielsweise verschmolzen werden.

Um den Mantel mithilfe des Kerns winden zu können, kann der Mantel zunächst um den Kern herumgelegt werden, wobei der Mantel sich insbesondere entlang der Umfangsrichtung des Kerns an diesen anschmiegen und diesen zumindest teilweise oder sogar vollständig umgeben kann.

Soll der Mantel einfach am Kern befestigt werden, können der Mantel und der Kern stoffschlüssig miteinander verbunden werden. Insbesondere wenn sowohl das Mantelmaterial als auch der Kern aus einem thermoplastischen Polymermaterial gefertigt sind, können der Mantel und der Kern unter Temperatur- und Druckeinwirkung einfach aneinander befestigt und zum Beispiel verschmolzen werden. Zum Beispiel kann der Mantel von einer Form gegen den Kern gedrückt werden, wobei die Form vorzugsweise nicht am Mantel haftet und beispielsweise Teflon- oder Keramikoberflächen aufweist, die den Mantel bei der Herstellung der Elektrode kontaktieren. Um den Mantel an dem Kern befestigen zu können, können Mantel und Kern auf bis zu 350°C und beispielsweise auf 200°C erwärmt werden. Diese Temperaturen können für bis zu 20 Minuten und beispielsweise fürs wenigstens 1 Sekunde aufrechterhalten werden. Das Material des Mantels und Kerns können bei einer derartigen Temperaturbehandlung miteinander verschmelzen und sich so nahtlos oder zumindest nahezu nahtlos stoffschlüssig miteinander verbinden.

Beispielsweise kann als Flüssigkristall-Polymer Polyurethan oder ein anderes Polymer verwendet werden.

Im um die zylindrische Form und/oder den Kern gewundenen Zustand kann der gewundene Verbindungsabschnitt erwärmt werden. Beispielsweise kann diese Erwärmung oder Temperierung kurzzeitig sein und etwa eine 1 Sekunde betragen. Der Erwärmungsvorgang kann jedoch durchaus mehrere Minuten und beispielsweise bis zu 10 Minuten, bis zu 30 Minuten oder sogar bis zu 60 Minuten andauern. Temperaturen des gewundenen Verbindungsabschnitts können bei der thermischen Behandlung bis zu 150°C oder sogar bis zu 250°C oder sogar bis zu 350°C betragen.

Beispielsweise kann die Erwärmung mithilfe eines Heißluftstroms erfolgen, der auf die Außenseite des gewundenen Verbindungsabschnitts gerichtet ist. Hierdurch kann die Außenseite stärker erwärmt werden als die Innenseite, insbesondere wenn die Erwärmung nur für eine kurze Zeit, also wenige beispielsweise bis zu 60 Sekunden, anhält.

Alternativ kann der Verbindungsabschnitt und insbesondere dessen Mantel gegen eine Kante drückend über die Kante gezogen werden, um die Außenseite dauerhaft zu strecken.

Der Verbindungsabschnitt und insbesondere dessen Mantel liegt dabei vorzugsweise mit seiner Innenseite an der Kante an. Zusätzlich zur Formgebung mithilfe der Kante kann der Verbindungsabschnitt erwärmt werden. So kann die Kante Teil eines erwärmbaren Werkzeugs sein. Alternativ kann der Heißluftstrom auf Teile des Verbindungsabschnitts gerichtet werden. Insbesondere kann der Heißluftstrom auf noch über die Kante zu ziehende oder bereits an der Kante anliegende Teile des Verbindungsabschnitts gerichtet werden.

Alternativ oder zusätzlich zur mechanischen und/oder thermischen Formgebung des gewundenen Verbindungsabschnitts kann der Verbindungsabschnitt zumindest einseitig mit einer mechanische Spannung auf den Leiterabschnitt ausübenden Schicht versehen werden. Die Spannung kann eine Druckspannung oder eine Zugspannung sein. Ist die mechanische Spannung eine Druckspannung, so windet die Schicht den Verbindungsabschnitt so, dass die beschichtete Seite die Außenseite des Verbindungsabschnitts ausformt. Ist die mechanische Spannung eine Zugspannung, so windet die Schicht den Verbindungsabschnitt so, dass die beschichtete Seite die Innenseite bildet. Die Spannung windet also den Verbindungsabschnitt und insbesondere dessen Mantel.

Jede der Verbindungsleitungen kann mit einem separaten Kontaktelement elektrisch leitfähig verbunden sein. Es kann ausreichen, dass die Elektrode zwei oder drei Verbindungsleitungen und Kontaktelemente aufweist, beispielsweise wenn die Elektrode zum Verbinden eines Herzschrittmachers oder Defibrillators mit dem Herzen ausgebildet ist. Alternativ kann die Elektrode mehr als drei und beispielsweise bis zu 10, 20, 30, 40, 50 oder sogar mehr als 50 Verbindungsleitungen und Kontaktelemente aufweisen.

Die erfindungsgemäße Elektrode kann nach dem erfindungsgemäßen Verfahren hergestellt sein. Mit dem erfindungsgemäßen Verfahren kann die erfindungsgemäße Elektrode hergestellt werden.

Im Folgenden ist die Erfindung beispielhaft anhand von Ausführungsformen mit Bezug auf die Zeichnungen erläutert. Die unterschiedlichen Merkmale der Ausführungsformen können dabei unabhängig voneinander kombiniert werden, wie es bei den vorteilhaften Ausgestaltungen bereits dargelegt wurde. Es zeigen:
- Fig. 1: eine schematische Perspektivdarstellung eines Ausführungsbeispiels der erfindungsgemäßen implantierbaren Elektrode,
- Fig. 2 bis 4: schematische Darstellungen eines Ausführungsbeispiels eines Kontaktabschnitts der erfindungsgemäßen Elektrode,
- Fig. 5 und 6: ein Vorprodukt eines Ausführungsbeispiels der implantierbaren Elektrode,
- Fig. 7: das Vorprodukt der Figuren 5 und 6 mit einem Streckungswerkzeug,
- Fig. 8 und 9: zwei Ausführungsbeispiele von Verbindungsabschnitten der erfindungsgemäßen Elektrode,
- Fig. 10: eine schematische Darstellung eines Ausführungsbeispiels des erfindungsmäßen Verfahrens zum Herstellen der implantierbaren Elektrode.

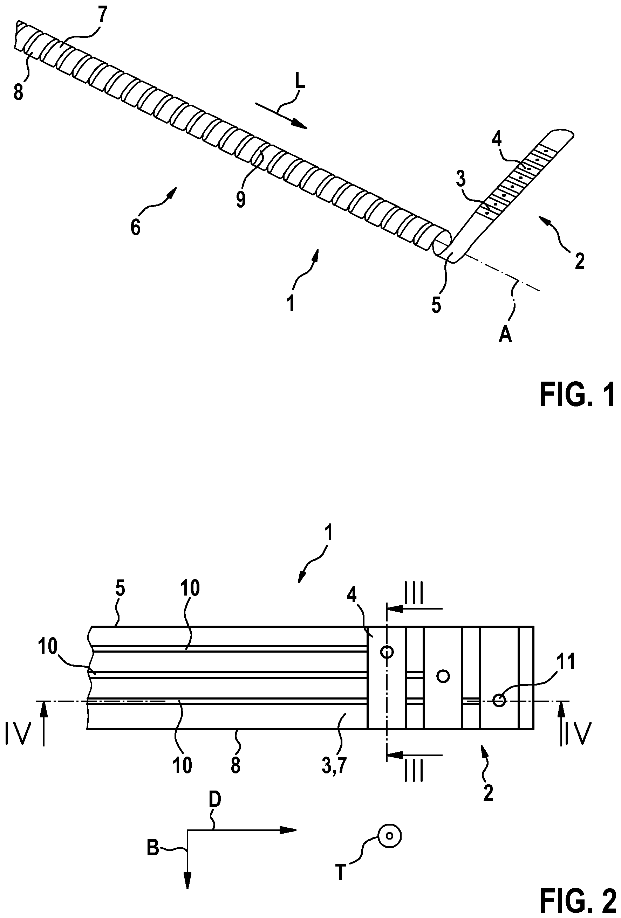

Zunächst sind Aufbau und Funktion einer erfindungsgemäßen implantierbaren Elektrode mit Bezug auf das Ausführungsbeispiel der Figur 1 beschrieben

Figur 1 zeigt die implantierbare Elektrode 1 schematisch in einer perspektivischen Ansicht. Insbesondere zeigt die Figur 1 ein distales Ende der Elektrode 1. Das distale Ende ist als ein Kontaktabschnitt 2 mit einer Kontaktseite 3 ausgebildet. Zumindest auf der Kontaktseite 3 weist der Kontaktabschnitt 2 wenigstens ein Kontaktelement 4 auf, das im implantierten Zustand der Elektrode 1 Körpergewebe und beispielsweise Muskel- oder Nervenzellen elektrisch kontaktiert. In der Figur 1 ist die Elektrode 1 beispielhaft mit acht Kontaktelementen 4 versehen, die alle auf der einen Kontaktseite 3 angeordnet sind.

Die Kontaktelemente 4 sind auf einem Mantel 5 der Elektrode 1 angeordnet. Innerhalb des Mantels 5 befinden sich Verbindungsleitungen, die elektrisch leitfähig mit den Kontaktelementen 4 verbunden sind. Da die Verbindungsleitungen in den Mantel 5 eingebettet sind, sind diese in der Figur 1 nicht dargestellt. Die Verbindungsleitungen dienen zur elektrischen Verbindung der Kontaktelemente 4 mit einem medizinischen Gerät, zum Beispiel ein Schrittmacher oder ein Defibrillator, und erstrecken sich durch die Elektrode 1 und insbesondere durch den Mantel 5. Ein sich an den Kontaktabschnitt 3 anschließender Verbindungsabschnitt 6 der Elektrode 1 führt die Verbindungsleitungen zum medizinischen Gerät. Weist die Elektrode 1 ein dem distalen Ende gegenüberliegendes Anschlussende auf, so ist der Verbindungsabschnitt 6 zwischen dem distalen Ende und dem Anschlussende angeordnet. Das Anschlussende dient zum Anschluss der Elektrode 1 an das medizinische Gerät, beispielsweise ein Defibrillator, ein Herzschrittmacher oder ein anderes medizinisches Gerät, etwa zur Nervenstimulation.

Der Verbindungsabschnitt 6 ist gewunden und mit einer Vielzahl von Windungen dargestellt. Der Kontaktabschnitt 2 hingegen kann ungewunden und insbesondere eben ausgebildet sein. Aufgrund der gewundenen Form des Verbindungsabschnitts 6 ist dieser entlang einer Längsrichtung L des Verbindungsabschnitts 6 dehnbar, also in die Länge ziehbar. Entlang der Längsrichtung L erstreckt sich eine Längsachse A des Verbindungsabschnitts 6, wobei der Mantel 5 um die Längsachse herum gewunden ist. Da die Längsachse A von den Windungen des Verbindungsabschnitts 6 umgeben ist, ist diese der Einfachheit halber nur teilweise dargestellt.

Der Verbindungsabschnitt 6 und insbesondere dessen Windungen weisen eine Außenseite 7 auf, die von der Längsachse A weg weist und die parallel zur Längsrichtung L ausgerichtet sein kann. Ist der Mantel 5 des Verbindungsabschnitts 6 oder der gesamten Elektrode 1 flächig ausgebildet, so kann die Außenseite 7 eine breite Seite des Verbindungsabschnitts 6 sein. Eine Schmalseite 8 des gewundenen Mantels 5 kann in oder entgegen der Längsrichtung L weisend angeordnet sein. Die Windungen können in der Längsrichtung L hintereinander angeordnet sein und im Wesentlichen miteinander fluchten, sodass die Windungen quer zur Längsrichtung L nicht nennenswert über andere der Windungen überstehen.

Gegenüber der Außenseite 7 kann der gewundene Mantel 5 des Verbindungsabschnitts 6 eine Innenseite 9 aufweisen, die der Längsachse A zugewandt ist. Die Innenseite 9 kann wie die Außenseite 7 im Wesentlichen eben ausgebildet sein, sodass zumindest im Bereich des Verbindungsabschnitts 6 oder im Bereich der gesamten Elektrode 1 der Mantel 5 als ein flaches Band ausgeformt ist. Ist die Innenseite 9 eben oder glatt und sich entlang der Längsachse A erstreckend ausgebildet, kann der Verbindungsabschnitt 6 einfach mithilfe eines Führungsdrahtes implantiert werden.

Die Kontaktseite 3 ist im Ausführungsbeispiel der Figur 1 auf der Außenseite 7 vorgesehen. Alternativ kann die Kontaktseite 3 auf der Innenseite 9 angeordnet sein. Ferner können sowohl auf der Außenseite 7 als auch auf der Innenseite 9 Kontaktelemente 4 ausgebildet sein, wenn der Kontaktabschnitt 2 beidseitig Gewebe kontaktieren soll.

Aufgrund der mehreren Windungen des Verbindungsabschnitts 6 kann der Verbindungsabschnitt 6 und insbesondere dessen Mantel 5 helixförmig ausgebildet sein.

Im Ausführungsbeispiel der Figur 1 erstreckt sich der Kontaktabschnitt 2 im Wesentlichen senkrecht zur Längsrichtung L vom Verbindungsabschnitt 6 weg. Alternativ kann sich der Kontaktabschnitt 2 jedoch auch in einer anderen als der dargestellten Richtung und beispielsweise parallel zur Längsrichtung L erstrecken.

Figur 2 zeigt einen flächigen Mantel 5 als Halbfabrikat des Mantels 5 eines weiteren Ausführungsbeispiels der erfindungsgemäßen Elektrode 1. Für Elemente, die in Funktion und/oder Aufbau Elementen des Ausführungsbeispiels der Figur 1 entsprechen, sind dieselben Bezugszeichen verwendet. Im Folgenden ist lediglich auf die Unterschiede zum Ausführungsbeispiel der Figur 1 eingegangen.

Die Außenseite 7 des flächigen Mantels 5 entspricht der Außenseite 7 des gewundenen Verbindungsabschnitts 6. Auf der Außenseite 7 des flächigen Mantels 5 sind drei streifenförmige Kontaktelemente 4 angeordnet, die in einer Längsrichtung D des Kontaktabschnitts 2 hintereinander und beabstandet voneinander angeordnet sind. In einer Breitenrichtung B des Kontaktabschnitts 2 können die Kontaktelemente 5 die Außenseite 7 vollständig bedecken.

Jedes der Kontaktelemente 4 kann mit einer anderen Verbindungsleitung 10 der Elektrode 1 elektrisch leitfähig verbunden sein. Somit kann die Elektrode 1 beispielsweise drei Verbindungsleitungen 10 aufweisen. Jede der Verbindungsleitungen 10 kann mithilfe einer anderen Durchkontaktierung 11 mit jeweils einem der Kontaktelemente 4 elektrisch leitfähig verbunden sein.

Im Ausführungsbeispiel der Figur 2 erstrecken sich die Verbindungsleitungen 10 nur bis zu der Durchkontaktierung 11, welche die entsprechende Verbindungsleitung 10 mit dem jeweiligen Kontaktelement 4 elektrisch leitfähig verbindet. Alternativ können sich die Verbindungsleitungen 10 jedoch auch in der Längsrichtung D bis hinter diese Durchkontaktierung 11 und sich sogar vollständig durch den Kontaktabschnitt 2 erstrecken. Eine ungewollte Kontaktierung einer der Verbindungsleitungen 10 mit einem anderen Kontaktelement 4 ist dadurch verhindert, dass das Kontaktelement 4 auf der Außenseite 7 und die Verbindungsleitungen 10 in einer quer zur Längsrichtung D und zur Breitenrichtung B weisenden Dickenrichtung T beabstandet von der Außenseite 7 angeordnet sind.

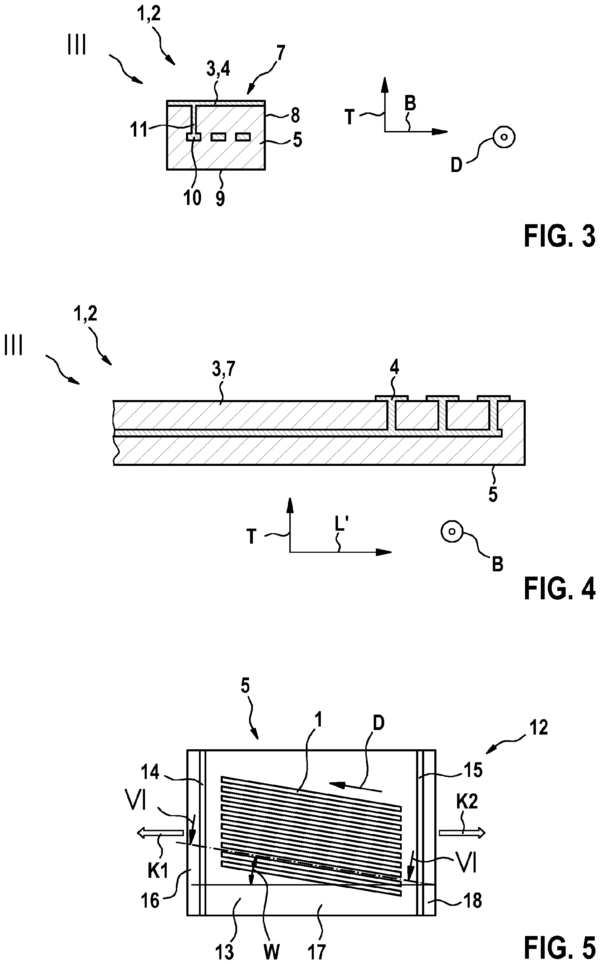

Figur 3 zeigt den Kontaktabschnitt 2 des Ausführungsbeispiels der Figur 2 schematisch in einer Schnittansicht III, die sich quer zur Längsrichtung D durch eines der Kontaktelemente 4 erstreckt. Die Längsrichtung D weist aus der Zeichenebene heraus. In der Breitenrichtung B sind die Verbindungsleitungen 10 nebeneinander und beabstandet zueinander angeordnet. In der Dickenrichtung T sind die Verbindungsleitungen 10 beabstandet zum Kontaktelement 4 vorgesehen. Die Durchkontaktierung 11, die das dargestellte Kontaktelement 4 mit einer der Verbindungsleitungen 10 elektrisch leitfähig verbindet, verläuft in der Dickenrichtung T von der Verbindungsleitung 10 zum Kontaktelement 4.

Die Verbindungsleitungen 10 können auf der der Außenseite 7 gegenüberliegenden Innenseite 9 des Mantels 5 angeordnet sein. Im Ausführungsbeispiel der Figur 3 sind die Verbindungsleitungen 10 jedoch so in den Mantel 5 eingebettet, dass die Verbindungsleitungen 10 quer zur Längsrichtung D nur über eines der Kontaktelemente 4 und die jeweilige Durchkontaktierung 11 kontaktierbar sind.

Figur 4 zeigt den Kontaktabschnitt 2 des Ausführungsbeispiels der Figuren 2 und 3 in einer Schnittansicht IV, wobei die Schnittebene durch eine der Verbindungsleitungen 10, also parallel zur Längsrichtung D und zur Dickenrichtung T, verläuft.

Figur 5 zeigt ein Halbfabrikat 12 mit mehreren implantierbaren Elektroden 1 in einer schematischen Aufsicht. Das Halbfabrikat 12 kann weniger als die dargestellten Elektroden 1 und beispielsweis mindestens eine Elektrode 1 aufweisen. Die Verbindungsleitungen 10 sind zwischen zwei den Mantel 5 ausbildenden Folien 13 angeordnet, wobei in der Ansicht der Figur 5 eine der Folien von der Folie 13 verdeckt ist. Die verdeckte Folie wird mechanisch gestreckt, was durch die Kraftpfeile K1, K2 dargestellt ist. Die Kraftpfeile K1, K2 der auf die verdeckt dargestellte Folie einwirkenden Streckkraft weisen voneinander weg, sodass die verdeckte Folie auseinandergezogen, also gestreckt wird. Die gestreckte Folie kann später die Außenseite 7 der Elektrode 1 ausbilden. Auf die Folie 13 wirkt die Streckkraft nicht direkt ein, sodass die Folie 13 nicht oder zumindest weniger gestreckt wird als die verdeckte Folie.

Um die verdeckte Folie mechanisch besser von der Folie 13 entkoppeln zu können, kann das Halbfabrikat 12 mit quer zu den Kraftpfeilen K1, K2 verlaufenden Nuten 14, 15 versehen sein. Die Nuten 14, 15 können die Folie 13 in unterschiedliche Abschnitte 16, 17, 18, die parallel zu den Nuten 14, 15 verlaufen, teilen. Der zwischen den äußeren Abschnitten 16 und 18 angeordnete mittlere Abschnitt 17 weist die Elektroden 1 auf. Die Elektroden 1 können sich nicht bis auf die äußeren Abschnitte 16, 18 erstrecken.

Um zu gewährleisten, dass die Elektroden 1 nach der Vereinzelung helixförmig und nicht lediglich spiralförmig ineinander gewickelt sind, erstrecken sich die Elektroden 1 unter einem Winkel W zu den Kraftpfeilen K1, K2. Der Wert für den Winkel W liegt vorzugsweise zwischen 10 und 60 Grad. Folglich erstreckt sich die Längsrichtung D unter diesem Winkel W zur wirkenden Streckkraft. Der Winkel W kann im Wesentlichen dem Steigungswinkel der Windungen des helixförmigen Verbindungsabschnitts 6 entsprechen.

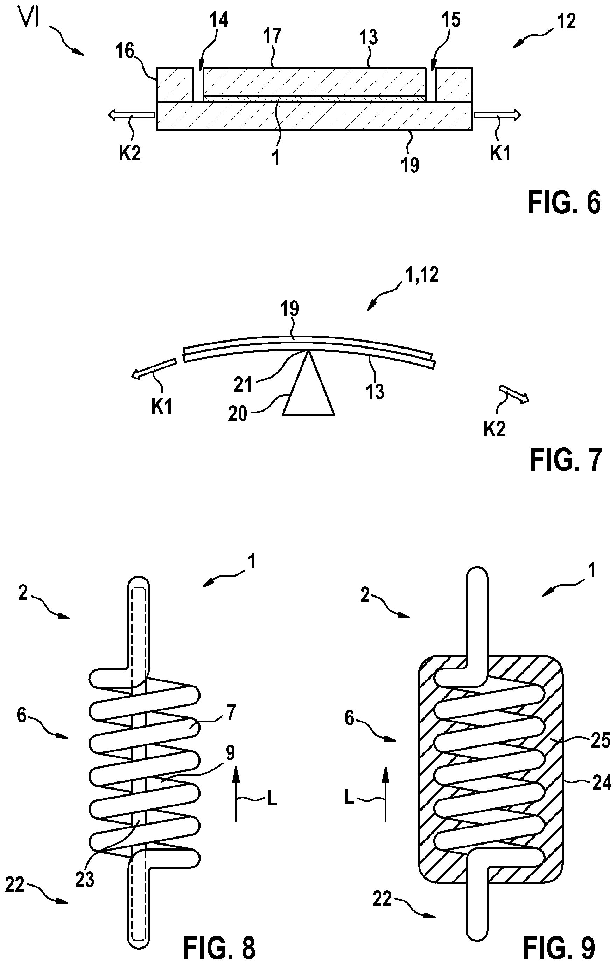

Figur 6 zeigt das Ausführungsbeispiel der Figur 5 schematisch in einer Schnittansicht entlang der Linie VI in der Figur 5. Wie aus der Figur 6 ersichtlich, können sich die Nuten 14,

15 vollständig durch die Folie 13 erstrecken, um deren Abschnitte 16, 17, 18 vollständig voneinander zu trennen. In der Ansicht der Figur 6 ist auch die Folie 19 erkennbar. Die Folie 19 wird, wie durch die Kraftpfeile K1, K2 dargestellt, durch die Streckkraft stärker gestreckt als die Folie 13. Die Streckung erfolgt plastisch. Aufgrund der Streckung krümmen sich die Elektroden 1 und insbesondere deren Verbindungsabschnitte 6 so, dass die Verbindungsabschnitte 6 der Elektroden 1 helixförmig ausgeformt sind.

Figur 7 zeigt ein weiteres Ausführungsbeispiel einer Elektrode 1 oder eines Halbfabrikats 12 während der mechanischen Streckung der Folie 19. Die Elektrode 1 oder das Halbfabrikat 12 wird über ein Streckwerkzeug 20 gezogen, um die Folie 19 stärker zu strecken als die Folie 13. Dabei wird die Folie 13 gegen das Streckwerkzeug 20 und insbesondere gegen eine Kante 21 des Streckwerkzeuges 20 drückend über das Streckwerkzeug 20 gezogen. Dies ist beispielsweise durch die Kraftpfeile K1, K2 dargestellt, die insbesondere hier als Kraftvektoren aufzufassen sind. Die Kraftvektoren K1, K2 weisen Komponenten auf, die auf die Kante 21 zu weisen, die Elektrode 1 bzw. das Halbfabrikat 12 also auf die Kante 21 zu ziehen. Ferner ist der Kraftvektor K1 größer als der Kraftvektor K2, sodass die Elektrode 1 beziehungsweise das Halbfabrikat 12 im Wesentlichen in Richtung des Kraftvektors K1 über die Kante 21 gezogen werden.

Figur 8 zeigt ein weiteres Ausführungsbeispiel der implantierbaren Elektrode 1 in einer schematischen Seitenansicht. Für Elemente, die in Funktion und/oder Aufbau Elementen der bisherigen Ausführungsbeispiele entsprechen, sind dieselben Bezugszeichen verwendet. Der Kürze halber ist im Folgenden lediglich auf die Unterschiede zu den bisherigen Ausführungsbeispielen eingegangen.

Der Verbindungsabschnitt 6 ist wie bei den bisherigen Ausführungsbeispielen gewendelt, also beispielsweise helixförmig dargestellt. Ferner ist die Elektrode 1 des Ausführungsbeispiels der Figur 8 mit dem Kontaktabschnitt 2 dargestellt, der sich parallel zur Längsrichtung L erstreckt. Darüber hinaus ist die implantierbare Elektrode 1 mit einem Anschlussabschnitt 22 zum Anschließen der Elektrode 1 an ein medizinisches Gerät gezeigt.

Ferner ist die Elektrode 1 mit einem Kern 23 dargestellt. Der Kern 23 kann zur Herstellung der Wendelung des Verbindungsabschnitts 6 vorgesehen sein. Beispielsweise kann der Mantel 5 im Bereich des Verbindungsabschnitts 6 um den Kern 23 herum gewickelt werden, sodass der Kern 23 als eine Wickelform bereit gestellt sein kann. Nach der Ausformung des gewendelten Verbindungsabschnitts 6 kann der Kern 23 im Verbindungsabschnitt 6 verbleiben. Alternativ kann der Kern 23 aus dem gewendelten Verbindungsabschnitt 6 entfernt werden. Beispielsweise wenn der Verbindungsabschnitt 6 auch ohne den Kern 23 dauerhaft gewendelt ausgeformt ist, kann es sein, dass der Kern 23 nicht mehr notwendig ist. Soll der Kern 23 jedoch im Verbindungsabschnitt 6 verbleiben, so kann es vorteilhaft sein, wenn der Kern 23 am Kontaktabschnitt 2, am Verbindungsabschnitt 6 und/oder am Anschlussabschnitt 22 unverschiebbar befestigt ist. Quer und entlang der Längsrichtung L kann der Kern 23 flexibel ausgebildet sein, um ein Verbiegen und ein Strecken der Elektrode 1 quer und entlang der Längsrichtung L zu ermöglichen.

Um den Verbindungsabschnitt 6 mithilfe des Kerns 23 oder auch ohne den Kern 23 helixförmig ausbilden zu können, kann der Verbindungsabschnitt 6 in die gezeigte Form gewickelt und dann thermisch behandelt und insbesondere erwärmt werden. Durch die Erwärmung reduzieren sich Spannungen in dem mechanisch gewundenen und gehaltenen Verbindungsabschnitt 6. Nach einem Abkühlen des mechanisch gewundenen Verbindungsabschnitts 6 kann dieser in der gewundenen Form verbleiben.

Figur 9 zeigt ein weiteres Ausführungsbeispiel der erfindungsgemäßen Elektrode 1. Für Elemente, die in Form und/oder Funktion Elementen der bisherigen Ausführungsbeispiele entsprechen, sind dieselben Bezugszeichen verwendet. Der Kürze halber ist im Folgenden lediglich auf die Unterschiede zum Ausführungsbeispiel der Figur 6 eingegangen.

Die Elektrode 1 ist ohne den optional womöglich vorhandenen Kern 23 dargestellt. Ferner ist der Verbindungsabschnitt 6 in einem Körper 24 angeordnet, der um den Verbindungsabschnitt 6 und womöglich um an den Verbindungsabschnitt 6 angrenzende Teile des Kontaktabschnitts 2 und/oder des Anschlussabschnitts 3 herum gespritzt oder gegossen sein kann. Beispielsweise kann der Körper 24 aus einer Vergussmasse, etwa Silikon, gefertigt sein. Der Körper 24 ist zumindest in der Längsrichtung L vorzugsweise elastisch, sodass er ein Strecken des Verbindungsabschnitts 6 entlang der Längsrichtung L ermöglicht. Der Körper 24 kann die Implantation der Elektrode 1 vereinfachen, da er eine glatte Außenseite 25, die entlang der Längsrichtung L verläuft, bereitstellt.

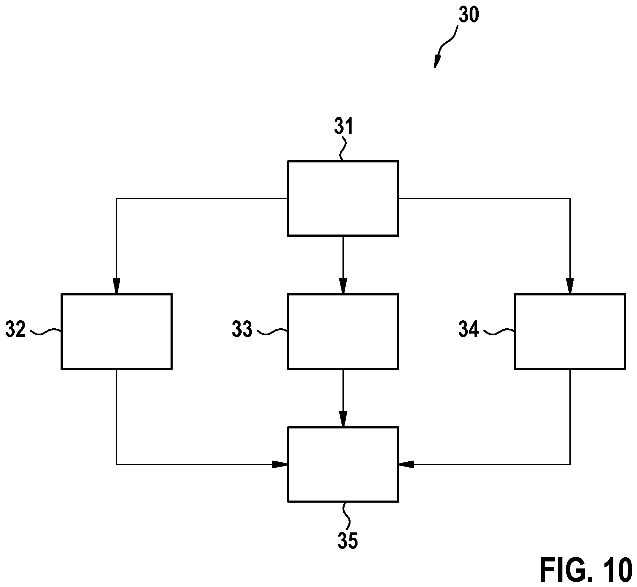

Figur 10 zeigt das erfindungsgemäße Verfahren zum Herstellen der implantierbaren Elektrode 1 schematisch als ein Flussdiagram. Für Elemente der bisherigen Ausführungsbeispiele, die im Folgenden zur Erläuterung des Verfahrens hilfreich sind, sind dieselben Bezugszeichen verwendet.

Das Verfahren 30 startet mit einem ersten Verfahrensschritt 31. Im ersten Verfahrensschnitt 31 kann beispielsweise eine Folie 13, 19 bereitgestellt werden, wobei die Folie 13, 19 den Mantel 5 wenigstens des Verbindungsabschnitts 6 oder der gesamten Elektrode 1 zumindest teilweise ausbildet. Um zumindest eine Verbindungsleitung 10 mit wenigstens einem Kontaktelement 4 elektrisch leitfähig verbinden zu können, kann im Verfahrensschritt 31 die Folie 13, 19 an einer vorbestimmten Position gelocht werden. Beispielsweise kann die Folie 13, 19 mechanisch, etwa mit einem Stanzwerkzeug, mithilfe eines Lasers oder einer Lithografievorrichtung gelocht werden.

Im Verfahrensschritt 31 kann ferner das Kontaktelement 4, die Verbindungsleitung 10 und die das Kontaktelement 4 und die Verbindungsleitung 10 verbindende Durchkontaktierung 11 an der Folie 13, 19 ausgebildet werden. Beispielsweise kann zunächst das Kontaktelement 4 oder die Verbindungsleitung 10 additiv oder subtraktiv auf einer Seite der Folie 13, 19 vorgesehen werden. Im Anschluss daran kann die Durchkontaktierung 11 in dem ausgebildeten Loch ausgeformt werden. Im Anschluss daran kann das bisher nicht ausgebildete Kontaktelement 4 oder die bisher nicht ausgebildete Verbindungsleitung 10 auf der anderen Seite der Folie 13, 19 ausgeformt werden. Das Kontaktelement 4 wird auf einer Seite und die Verbindungsleitung 10 auf einer dem Kontaktelement 4 gegenüberliegenden Seite der Folie 13, 19 ausgebildet. Das Loch verbindet die beiden Seiten miteinander.

Darüber hinaus kann im Verfahrensschritt 31 auf die Seite der Folie 13, 19, auf der die Verbindungsleitung 6 angeordnet ist, eine weitere Folie 13, 19 aufgebracht werden, sodass die Verbindungsleitung 6 nicht nur teilweise in die eine Folie 19, sondern vollständig zwischen den Folien 13, 19 in den Mantel 4 eingebettet ist. Die weitere Folie 13 kann eine Innenseite des Mantels 4 ausbilden.

Auf den Verfahrensschritt 31 folgt ein Verfahrensschritt, in dem zumindest der Verbindungsabschnitt 6 der Elektrode 1 gewendelt und beispielsweise helixförmig ausgeformt wird. Im auf den Verfahrensschritt 31 womöglich folgenden Verfahrensschritt 32 wird der Verbindungsabschnitt 6 einseitig stärker gestreckt als auf einer anderen, insbesondere gegenüberliegenden Seite. Optional kann zumindest die stärker gestreckte Seite des Verbindungsabschnitts 6 dabei erwärmt werden.

Die Streckung kann durch Ziehen an der stärker zu streckenden Seite und beispielsweise an der Folie 19 erfolgen. Optional kann die Elektrode 1 und insbesondere deren Verbindungsabschnitt 6 etwa mit der Folie 13 gegen ein Streckwerkzeug 20 gedrückt und dabei über das Streckwerkzeug 20 gezogen werden, um zumindest den Verbindungsabschnitt 6 einseitig stärker zu strecken. Ferner kann zumindest der Verbindungsabschnitt 6 in eine hohlzylindrische Form gebracht und hierzu beispielsweise um den Kern 23 herum gewickelt werden. In der hohlzylindrischen Form kann der Verbindungsabschnitt 6 gehalten und getempert werden, um durch die Wicklung des Verbindungsabschnitts 6 entstandene Spannungen zu entspannen. Danach kann der Verbindungsabschnitt 6 beziehungsweise die Elektrode 1 abgekühlt werden. Nach der Abkühlung verbleibt zumindest der Verbindungsabschnitt 6 in der gewickelten oder gewundenen Form.

Optional zum Verfahrensschritt 32 kann auch auf den Verfahrensschritt 31 der Verfahrensschritt 33 folgen. Im Verfahrensschritt 33 wird zumindest der Verbindungsabschnitt 6 der Elektrode 1 helixförmig gewickelt und dann in der helixförmigen Form fixiert, zum Beispiel durch Befestigung am Kern 23.

Alternativ oder zusätzlich zu einem der Verfahrensschritte 32 und 33 kann auf den Verfahrensschritt 31 der Verfahrensschritt 34 folgen. Im Verfahrensschritt 34 wird zumindest eine Seite, beispielsweise die Außenseite 7 und/oder die Innenseite 9 des Mantels 5, also zum Beispiel die Folie 19 oder die Folie 13, jeweils mit einer Beschichtung versehen, wobei die Beschichtung eine mechanische Spannung auf den Mantel des Verbindungsabschnitts 6 ausübt. Aufgrund der mechanischen Spannung krümmt sich der Verbindungsabschnitt 6 in die Helixform. Ist die Schicht auf der Außenseite 7 angeordnet, so kann diese eine Druckspannung ausüben. Eine auf der Innenseite 9 angeordnete Schicht kann eine Zugspannung ausüben.

Auf jeden der Verfahrensschritte 32, 33, 34 kann der Verfahrensschritt 35 folgen, in den das Verfahren 30 endet.

### Bezugszeichenliste

- 1: implantierbare Elektrode
- 2: Kontaktabschnitt
- 3: Kontaktseite
- 4: Kontaktelement
- 5: Mantel
- 6: Verbindungsabschnitt
- 7: Außenseite
- 8: Schmalseite
- 9: Innenseite
- 10: Verbindungsleitung
- 11: Durchkontaktierung
- 12: Halbfabrikat
- 13: Folie
- 14, 15: Nut
- 16, 17, 18: Abschnitte der Folie
- 19: Folie, gestreckt
- 20: Streckwerkzeug
- 21: Kante
- 22: Anschlussabschnitt
- 23: Kern
- 24: Körper
- 25: Außenseite des Körpers
- 30: Verfahren
- 31: Start
- 32: Strecken
- 33: Wickeln und optional Befestigen
- 34: Beschichten
- 35: Ende
- A: Längsachse
- B: Breitenrichtung
- D: Längsrichtung des Kontaktabschnitts
- K1, K2: Kraftpfeil
- L: Längsrichtung des Verbindungsabschnitts
- W: Winkel

## Patentansprüche

1. Implantierbare Elektrode (1), umfassend:
ein Kontaktelement (4) zur Kontaktierung von Körpergewebe,
einen Verbindungsabschnitt (6) mit einer Verbindungsleitung (10) zur Verbindung eines medizinischen Geräts mit dem Kontaktelement (4),
einen zylindrischen Kern (23),
**dadurch gekennzeichnet, dass**
der Kern (23) entlang der Längsachse (A) der Elektrode flexibel ist und
der Verbindungsabschnitt (6) um den zylindrischen Kern (23) herum gewunden ist und einzelne oder alle Windungen am Kern befestigt sind, wobei der Verbindungsabschnitt (6) flächig ausgebildet ist und die zumindest eine Verbindungsleitung (10) in einen flächigen Mantel (5) eingebettet ist.

2. Implantierbare Elektrode nach Anspruch 1, **dadurch gekennzeichnet, dass** der Verbindungsabschnitt (6) zumindest abschnittsweise helixförmig ausgebildet ist.

3. Implantierbare Elektrode nach Anspruch 1 oder 2, **dadurch gekennzeichnet, dass** der Verbindungsabschnitt (6) als ein um die Längsachse (A) herum gewundenes flaches Band ausgeformt ist.

4. Implantierbare Elektrode nach einem der Ansprüche 1 bis 3, **dadurch gekennzeichnet, dass** der Verbindungsabschnitt (6) breite und schmale Seiten aufweist, wobei eine der breiten Seiten eine von der Längsachse (A) weg weisende Außenseite (7) ist.

5. Implantierbare Elektrode nach Anspruch 4, **dadurch gekennzeichnet, dass** der Verbindungsabschnitt (6) eine der Längsachse (A) zugewandte Innenseite (9) aufweist, wobei die Innenseite (9) kleiner ist als die Außenseite (7).

6. Implantierbare Elektrode nach Anspruch 5, **dadurch gekennzeichnet, dass** die Außenseite (7) stärker gestreckt ist als die Innenseite (9).

7. Implantierbare Elektrode nach Anspruch 1, **dadurch gekennzeichnet, dass** der Mantel (5) als ein Flachbandkabel ausgebildet ist, wobei die zumindest eine Verbindungsleitung (10) zwischen zwei elektrisch isolierenden Folien angeordnet ist.

8. Implantierbare Elektrode (1) nach einem der Ansprüche 1 bis 7, **dadurch gekennzeichnet, dass** der Verbindungsabschnitt (6) mit einer eine mechanische Spannung auf den Verbindungsabschnitt (6) ausübenden Schicht versehen ist.

9. Implantierbare Elektrode nach einem der Ansprüche 1 bis 8, **dadurch gekennzeichnet, dass** die Elektrode (1) mit einem das Kontaktelement (4) aufweisenden Kontaktabschnitt (2) ausgestaltet ist, wobei der Kontaktabschnitt (2) im Wesentlich eben ausgebildet ist.

10. Verfahren (30) zum Herstellen einer implantierbaren Elektrode (1), umfassend:
ein Kontaktelement (4) zur Kontaktierung von Körpergewebe,
einen Verbindungsabschnitt (6) mit einer Verbindungsleitung (10) zur Verbindung eines medizinischen Geräts mit dem Kontaktelement (4),
einen zylindrischen Kern (23),
**dadurch gekennzeichnet, dass**
der Kern (23) entlang der Längsachse (A) der Elektrode flexibel ist und
der Verbindungsabschnitt (6) um den zylindrischen Kern (23) herum gewunden wird und einzelne oder alle Windungen am Kern befestigt werden (30, 31, 32, 33, 34),
wobei der Verbindungsabschnitt (6) flächig ausgebildet ist und die zumindest eine Verbindungsleitung (10) in einen flächigen Mantel (5) eingebettet ist.

11. Verfahren nach Anspruch 10, **dadurch gekennzeichnet, dass** der gewundene Verbindungsabschnitt (6) erwärmt wird (32, 33).

12. Verfahren nach einem der Ansprüche 10 bis 11, **dadurch gekennzeichnet, dass** der Verbindungsabschnitt (6) gegen ein Streckwerkzeug (20) drückend über das Streckwerkzeug (20) gezogen wird (32).

13. Verfahren nach einem der Ansprüche 10 bis 12, **dadurch gekennzeichnet, dass** der Verbindungsabschnitt (6) zumindest einseitig mit einer eine mechanische Spannung auf den Verbindungsabschnitt (6) ausübenden Schicht versehen wird (34).

## Claims

1. An implantable electrode (1) comprising:
a contact element (4) for making contact with body tissue;
a connecting section (6) comprising a connecting line (10) for connecting a medical device to the contact element (4);
a cylindrical core (23),
**characterized in that**
the core (23) is flexible along the longitudinal axis (A) of the electrode, and
the connecting section (6) is wound around the cylindrical core (23), and individual or all turns are attached to the core, wherein the connecting section (6) has a planar design, and the at least one connecting line (10) is embedded in a planar casing (5).

2. The implantable electrode according to claim 1, **characterized in that** the connecting section (6) has a helical design at least in some sections.

3. The implantable electrode according to claim 1 or 2, **characterized in that** the connecting section (6) is formed as a flat ribbon that is wound around the longitudinal axis (A).

4. The implantable electrode according to any one of claims 1 to 3, **characterized in that** the connecting section (6) has broad and narrow sides, wherein one of the broad sides is an outer side (7) pointing away from the longitudinal axis (A).

5. The implantable electrode according to claim 4, **characterized in that** the connecting section (6) has an inner side (9) facing the longitudinal axis (A), wherein the inner side (9) is smaller than the outer side (7).

6. The implantable electrode according to claim 5, **characterized in that** the outer side (7) is stretched to a greater extent than the inner side (9).

7. The implantable electrode according to claim 1, **characterized in that** the casing (5) is designed as a ribbon cable, wherein the at least one connecting line (10) is arranged between two electrically insulating films.

8. The implantable electrode (1) according to any one of claims 1 to 7, **characterized in that** the connecting section (6) is provided with a coating exerting mechanical stress onto the connecting section (6).

9. The implantable electrode according to any one of claims 1 to 8, **characterized in that** the electrode (1) is configured with a contact section (2) comprising the contact element (4), wherein the contact section (2) is substantially flat.

10. A method (30) for producing an implantable electrode (1), comprising:
a contact element (4) for making contact with body tissue;
a connecting section (6) comprising a connecting line (10) for connecting a medical device to the contact element (4);
a cylindrical core (23),
**characterized in that**
the core (23) is flexible along the longitudinal axis (A) of the electrode, and
the connecting section (6) is wound around the cylindrical core (23), and individual or all turns are attached to the core (30, 31, 32, 33, 34), wherein the connecting section (6) has a planar design, and the at least one connecting line (10) is embedded in a planar casing (5).

11. The method according to claim 10, **characterized in that** the wound connecting section (6) is heated (32, 33).

12. The method according to any one of claims 10 to 11, **characterized in that** the connecting section (6) is drawn (32) over a stretching tool (20) so as to press against the stretching tool (20).

13. The method according to any one of claims 10 to 12, **characterized in that** the connecting section (6) is provided (34) on at least one side with a coating exerting mechanical stress onto the connecting section (6).

## Revendications

1. Electrode implantable (1) comprenant :
un élément de contact (4) prévu pour la mise en contact avec le tissu corporel,
un segment de liaison (6) avec une ligne de liaison (10) pour la connexion d'un appareil médical avec l'élément de contact (4),
un noyau cylindrique (23),
**caractérisée en ce que**
le noyau (23) est flexible le long de l'axe longitudinal (A) de l'électrode et
le segment de liaison (6) est enroulé autour du noyau (23) cylindrique et certains ou tous les enroulements sont fixés sur le noyau, où le segment de liaison (6) est conçu plan et l'au moins une ligne d'électrode (10) est incorporée dans une gaine plane (5).

2. Electrode implantable selon la revendication 1, **caractérisée en ce que** le segment de liaison (6) est conçu au moins partiellement en forme d'hélice.

3. Electrode implantable selon la revendication 1 ou 2, **caractérisée en ce que** le segment de liaison (6) est moulé sous la forme d'une bande plate enroulée autour de l'axe longitudinal (A).

4. Electrode implantable selon l'une des revendications 1 à 3, **caractérisée en ce que** le segment de liaison (6) présente des côtés larges et étroits, où l'un des côtés larges est un côté extérieur (7) pointant au loin à partir de l'axe longitudinal (A).

5. Electrode implantable selon la revendication 4, **caractérisée en ce que** le segment de liaison (6) présente un côté intérieur (9) faisant face à l'axe longitudinal (A), où le côté intérieur (9) est plus petit que le côté extérieur (7).

6. Electrode implantable selon la revendication 5, **caractérisée en ce que** le côté extérieur (7) est davantage étiré que le côté intérieur (9).

7. Electrode implantable selon la revendication 1, **caractérisée en ce que** la gaine (5) est conçue sous la forme d'une limande, où l'au moins une ligne de liaison (10) est disposée entre deux feuilles isolantes électriquement.

8. Electrode implantable selon l'une des revendications 1 à 7, **caractérisée en ce que** le segment de liaison (6) est muni d'une couche exerçant une tension mécanique sur le segment de liaison (6).

9. Electrode implantable selon l'une des revendications 1 à 8, **caractérisée en ce que** l'électrode (1) est conçue avec un segment de contact (2) présentant l'élément de contact (4), où le segment de contact (2) est conçu essentiellement plan.

10. Procédé (30) de fabrication d'une électrode implantable (1), comprenant :
un élément de contact (4) prévu pour la mise en contact avec du tissu corporel,
un segment de liaison (6) avec une ligne de liaison (10) pour la connexion d'un appareil médical avec l'élément de contact (4),
un noyau cylindrique (23),
**caractérisée en ce que**
le noyau (23) est flexible le long de l'axe longitudinal (A) de l'électrode et
le segment de liaison (6) est enroulé autour du noyau (23) cylindrique et certains ou tous les enroulements sont fixés sur le noyau (30, 31, 32, 33, 34),
où le segment de liaison (6) est conçu plan et l'au moins une ligne d'électrode (10) est incorporée dans une gaine (5) plane.

11. Procédé selon la revendication 10, **caractérisé en ce que** le segment de liaison (6) est réchauffé (32, 33).

12. Procédé selon l'une des revendications 10 ou 11, **caractérisé en ce que** le segment de liaison (6) est étiré (32) pressé contre un outil d'extension (20) par le biais de l'outil d'extension (20).

13. Procédé selon l'une des revendications 10 à 12, **caractérisé en ce que** le segment de liaison (6) est muni d'une couche (34) exerçant au moins unilatéralement une tension mécanique sur le segment de liaison (6).
